Europäisches Patentamt

European Patent Office

Office européen des brevets

(11) Publication number: **0 239 048**
**A2**

## EUROPEAN PATENT APPLICATION

(21) Application number: **87104233.9**

(22) Date of filing: **23.03.87**

(51) Int. Cl.⁴: **A61B 17/36**

(30) Priority: **27.03.86 PL 258645**

(43) Date of publication of application:
**30.09.87 Bulletin 87/40**

(84) Designated Contracting States:
**AT DE GB IT**

(71) Applicant: **Politechnika Warszawska**
**Plac Jednosci Robotniczej 1**
**Warschau(PL)**

(72) Inventor: **Czachor, Marceli Stanislaw**
**ul. Oliwkowa 3**
**Warszawa(PL)**
Inventor: **Mikolajczak, Justyn**
**ul. Pieciolinii 7 m. 52**
**Warszawa(PL)**
Inventor: **Osinski, Roman Waclaw**
**ul. Rusznikarska 6 m. 7**
**Warszawa(PL)**
Inventor: **Szkup, Bogdan Ireneusz**
**ul. Nowotki 6 m. 96**
**Warszawa(PL)**
Inventor: **Wartanowicz, Tomasz**
**ul. Nowickiego 7 m. 22**
**Warszawa(PL)**

(74) Representative: **Martin, Jean-Jacques et al**
**Cabinet REGIMBEAU 26, Avenue Kléber**
**F-75116 Paris(FR)**

(54) **Low-temperature destroyer.**

(57) Low-temperature destroyer is provided with thermoelectric modules (5), whose cold junction is in thermal contact with core (2) of cryoprobe (1).

Thermoelectric modules (5) are connected in multi-stage thermoelectric cascades (4), which are arranged around the core (2) of cryoprobe (1). The cold junction of the highest stage of every cascade (4) is structurally connected with the core (2) of the cryoprobe (1), whereas the hot junction of the first stage of every cascade (4) is connected structurally with a cooling system (6). Moreover, to the core (2) of the cryoprobe (1) there is mounted a sorption pump (7) for maintaining inside the casing (3) a static vacuum, and in said casing (3) there is installed a vacuum valve (8).

FIG.1

## LOW-TEMPERATURE DESTROYER.

This invention relates to low-temperature destroyer used in cryosurgery, and particularly in neurosurgery, dermatology, ophthalmology and plastic surgery.

In low-temperature destroyers so far used cooling of cryoprobe tip is obtained by supplying to it either liquid nitrogen vapours, or employing Joule-Thomson effect observed during throttling of compressed gases, such as nitrogen monoxide and carbon dioxide. Nitrogen low-temperature destroyer consists of a container with liquid nitrogen, supply unit, control and measuring unit and cryoprobe. Liquid nitrogen is supplied under 45 kPa pressure through flexible siphon from the container into the cryoprobe and through its internal tube accommodated in a vacuum-tight casing to the cryoprobe tip, where nitrogen is separated and thus the cryoprobe tip temperature is lowered to the required level dependent upon nitrogen flow rate. However, this solution requires constant and systematic deliveries of hardly available liquid nitrogen, since its storage for a longer period of time is impossible. Gas low-temperature destroyer consists of a measuring and control unit, pressure vessel with a gas compressed to a pressure of 4.5 MPa, which is supplied through reducer and hose to cryoprobe and through its internal tube accommodated in a vacuum-tight casing to the cryoprobe tip, where the gas supplied to it is throttled and expanded which leads due to Joule-Thomson effect to lowering of temperature of the cryoprobe tip depending upon the gas flow rate. The gas having been used may not contain water vapour, since otherwise throttling nozzles in the cryoprobe tip would be covered with ice which could choke the tube.

Low-temperature destroyer known from the U.S. Patent Specification No. 4,519,389 is provided with a single thermocouple located in the cryoprobe tip, whose cold junction isolated electrically is the operating tip. The cold junction of thermocouple is cooled due to Peltier effect, but minimum obtainable temperatures don't exceed -20°C and obtainable freezing capabilities are very small. For this reason practical applicability of this invention is considerably limited.

The nature of the invention consists in that the presented low-temperature destroyer provided with at least one thermo-electric module, whose cold junction is in thermal contact with cryoprobe core has these modules connected in multistage thermoelectric cascades arranged around cryoprobe core. The cold junction of the highest stage of every cascade module is structurally connected with the core, and the hot junction of the first stage of every cascade module is structurally connected with the cooling system. Moreover, to the cryoprobe core there is mounted a sorption pump for maintaining a static vacuum inside the casing and in the casing there is installed a vacuum valve.

The solution according to the invention ensures miniaturization of dimensions and weight of low-temperature destroyer with simultaneous securing of low temperature of operating tip and high freezing capacity of the order of at least several Watts. This enables the low-temperature destroyer to be widely used in cryosurgery, and particularly in neurosurgery, dermatology, ophthalmology and plastic surgery. The presented design is featured with very simple operation and, which is particularly advantageous, the device is supplied with electric current only and the hot junctions of cascade module can be cooled with tap water. Tests have shown that the presented design ensures minimization of heat losses in spite of development of heat exchange area.

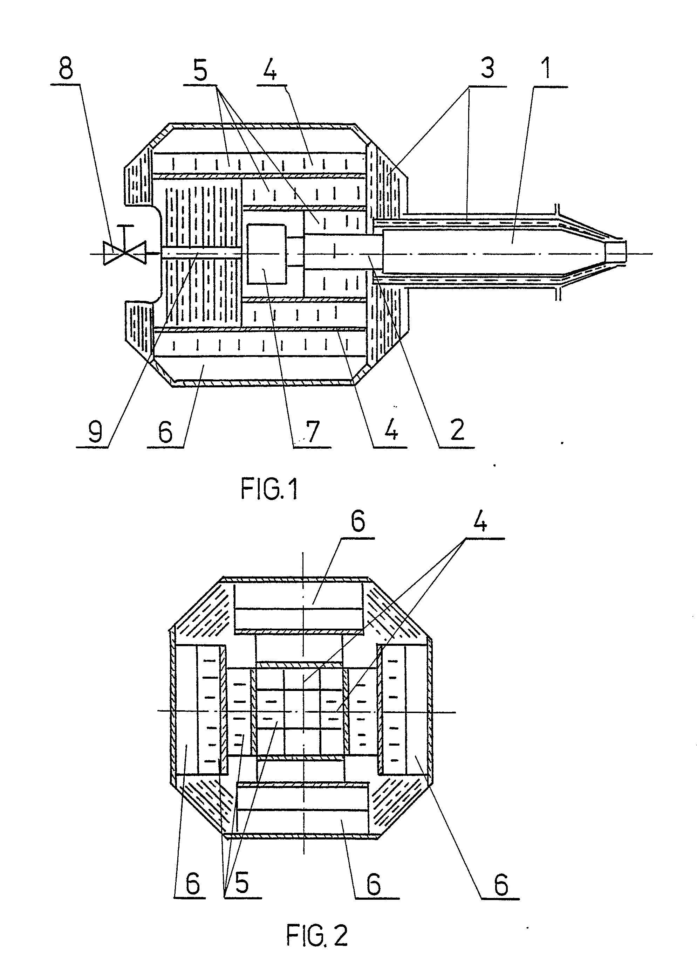

The subject of the invention has been shown on an example of embodiment presented on the accompanying drawing, wherein Fig. 1 presents longitudinal section of a simplified design of low-temperature destroyer, and Fig. 2 shows its cross section.

Low-temperature destroyer is provided with a cryoprobe 1, whose core 2 is mounted coaxially inside a vacuum-tight casing 3. Around said core 2 there are arranged four thermoelectric cascades 4, each of which consists of three thermoelectric modules 5 connected in multi-stade cascades. The cold junction of the thirds, that is most important stage of each cascade module 4 is in thermal contact with the core 2 of the cryoprobe 1, whereas the hot junction of the first stage of each cascade module is a water cooler 6. To the core 2 there is mounted a sorption pump 7. In the casing 3 there is a valve 8 connected with a capillary tube 9 enabling the device to be periodically degased. Due to the sorption pump 7 inside the casing 3 static vacuum is maintained at a level of $10^{-2}$ Pa.

Freezing effect of cryoprobe tip 1 is obtained by employing Peltier effect consisting in emission, or absorption of heat at the contact of two different conductors depending upon the direction of the flow of electric current through these conductors. Number modules and use of multi-stage thermoelectric cascades enable the temperature of the operating tip of the cryoprobe 1 to be lowered to the required level.

**Claims**

1. Low-temperature destroyer with cryoprobe employing Peltier effect characterized by thermo-electric modules (5) connected in thermoelectric multi-stage cascades (4) and arranged around core (2) of cryoprobe (1), the cold junction of the highest stage of every cascade (4) being connected structurally with the core (2) of cryoprobe (1) and the hot junction of the first stage of every cascade (4) being connected structurally with the cooling system (6) a sorption pump (7) being mounted to the core (2) of cryoprobe (1) for maintaining a static vacuum inside the casing (3) a vacuum valve (8) being mounted in said casing (3).

0 239 048

FIG.1

FIG. 2